# EUROPÄISCHE PATENTSCHRIFT

(11) **EP 0 568 882 B1**
(45) Veröffentlichungstag und Bekanntmachung des Hinweises auf die Patenterteilung: **14.07.1999**
(21) Anmeldenummer: 93106681.5
(22) Anmeldetag: 24.04.1993
(51) Int. Cl.: C07D 201/12, C07D 223/10

(54) **Verfahren zur Rückgewinnung von Caprolactam aus Polycaprolactam**
Process for the recovery of caprolactame from polycaprolactame
Procédé de récupération de caprolactame à partir de polycaprolactame

(30) Priorität: 07.05.1992 DE 4215087
(43) Veröffentlichungstag der Anmeldung: 10.11.1993
(73) Patentinhaber: BASF Aktiengesellschaft, 67063 Ludwigshafen (DE)
(72) Erfinder: Fuchs, Hugo, Dr., W-6700 Luwigshafen (DE); Ritz, Josef, Dr., W-6700 Luwigshafen (DE); Priester, Claus Ulrich, Dr., W-6700 Luwigshafen (DE); Neubauer, Gerald, Dr., W-6940 Weinheim (DE)

(56) Entgegenhaltungen:
- DD-A- 4 811
- DE-C- 850 614
- DE-C- 851 194
- DE-C- 851 195
- DE-C- 950 726
- US-A- 4 605 762
- PATENT ABSTRACTS OF JAPAN vol. 6, no. 094 (C-105)2. Juni 1982
- DATABASE WPI Week 8410, Derwent Publications Ltd., London, GB; AN 78-48679A
- DATABASE WPI Week 7127, Derwent Publications Ltd., London, GB; AN 71-46368S

## Beschreibung

Gegenstand der Erfindung ist ein Verfahren zur Rückgewinnung von Caprolactam aus Polycaprolactam durch hydrolytische Spaltung von Polycaprolactam mit Wasser.

Bei der Herstellung von Polycaprolactam sowie bei dessen Verarbeitung zu Formteilen wie Fäden, Fasern, Folien, spritzgegossenen oder extrudierten Formteilen fallen Polycaprolactamabfälle an, die entsorgt werden müssen. Desgleichen müssen die aus Polycaprolactam hergestellten Gebrauchsgüter, wie Folien, Gewebe, Verpackungen, geformte Teile letztendlich entsorgt werden. Hierfür bietet sich die Rückgewinnung von Caprolactam aus Polycaprolactam an.

Die DE-PS 850 614 beschreibt die Erhöhung der Caprolactam-Ausbeute bei der Depolymerisation von Polyamid-6 durch Zugabe geringer Mengen alkalisch wirkender Stoffe.

Aus DE-PS 851 195 ist bekannt, daß das hierbei erhaltene Caprolactam störende Nebenprodukte enthält, die durch einfache Rektifikation nicht zu entfernen sind. Gemäß DE-PS 950 726 erhält man bei der Depolymerisation von Polyamid-6 bei Arbeiten im Vakuum kein reines Lactam, sondern ein Rohlactam mit Verunreinigungen vornehmlich basischer Stoffe, und bei der Durchführung unter Druck kann man das gewonnene Rohlactam wegen des schlechten Reinheitsgrades nicht sofort wieder für eine erneute Polykondensation verwenden, sondern muß es erst auf umständliche und wenig wirtschaftliche Weise reinigen.

Aus der US-Patentschrift 4 605 762 wird bereits ein Verfahren beschrieben zur Depolymerisation von Kondensationspolymeren wie Polyestern, Polyamid-6,6 und Polycaprolactam durch hydrolytische Spaltung bei einer Temperatur von 200 bis 300°C unter erhöhtem Druck und Zuführung von Dampf. Aus der genannten US-Patentschrift ist nicht zu entnehmen, wie zu verfahren ist, um bei der hydrolytischen Spaltung von Polycaprolactam monomeres Caprolactam zu erhalten, das in die Reinigungsstufe bei der Herstellung von Caprolactam zurückgeführt werden kann.

Es war deshalb die technische Aufgabe gestellt, aus Polycaprolactam durch hydrolytische Spaltung monomeres Caprolactam zurückzugewinnen, das eine verbesserte Qualität hat und ohne Nachteil in die Reinigungsstufe bei der Caprolactamherstellung zurückgeführt werden kann.

Diese Aufgabe wird gelöst in einem Verfahren zur Rückgewinnung von Caprolactam aus Polycaprolactam durch hydrolytische Spaltung von Polycaprolactam mit 5 bis 50 Gewichtsteilen Wasser je Gewichtsteil Polycaprolactam unter Zusatz von Alkalihydroxid bei einem pH-Wert von 5 bis 10 einer Temperatur von 200 bis 350°C und einem Druck von 20 bis 100 bar unter Erhalt einer wäßrigen Lösung oder Suspension, die monomeres Caprolactam, dessen Oligomere und Polycaprolactam enthält und anschließendes Abtrennen von monomerem Caprolactam aus der wäßrigen Suspension durch Destillation oder Extraktion.

Das neue Verfahren hat den Vorteil, daß man Caprolactam mit verbesserter Qualität erhält, die es erlaubt, das so gewonnene monomere Caprolactam wiederum in die Reinigungsstufe bei der Caprolactamherstellung zurückzuführen. Weiter hat das neue Verfahren den Vorteil, daß es mit hoher Ausbeute verläuft und wenig nicht verwertbare Rückstände verbleiben.

Erfindungsgemäß geht man vom Polycaprolactam aus, das entsorgt werden soll oder von Abfällen, die bei der Polycaprolactamherstellung und dessen Verarbeitung zu Fäden, Folien, Spritzguß- oder Extrusionsteilen anfallen, ferner geformte Gebrauchsgegenstände wie Folien, Verpackungen, Gewebe, Fäden, extrudierte Teile, die entsorgt werden sollen. Zweckmäßig werden die zu spaltenden Polyamidgegenstände vor der hydrolytischen Spaltung zerkleinert, z.B. durch Mahlen, gegebenenfalls vorheriges Verdichten. Vorteilhaft geht man von Polycaprolactam in einer Teilchengröße von 1 bis 100 mm aus.

Für die hydrolytische Spaltung verwendet man je Gewichtsteil Polycaprolactam 5 bis 50, insbesondere 10 bis 20 Gewichtsteile Wasser. Die Umsetzung wird bei einer Temperatur von 200 bis 350°C durchgeführt. Vorteilhaft hält man eine Temperatur von 210 bis 300°C ein. Ferner wird die hydrolytische Spaltung unter einem Druck von 20 bis 100 bar durchgeführt. Es hat sich bewährt, den Druck durch Aufpressen eines inerten Schutzgases, wie Stickstoff zu erhöhen. Es versteht sich, daß Druck und Temperaturbedingungen so aufeinander abgestimmt sind, daß eine flüssige Phase vorliegt.

In der Regel hält man bei der hydrolytischen Spaltung Verweilzeiten von 3 bis 6 Stunden ein. Hierbei hat es sich bewährt, daß mindestens 60 Gew.-%, insbesondere 60 bis 90 % des eingesetzten Polycaprolactams in Caprolactam gespalten werden.

Die hydrolytische Spaltung wird unter Mitverwendung von Alkalihydroxiden wie Kaliumhydroxid oder insbesondere Natriumhydroxid bis zu einem pH-Wert bis 10 durchführt. Besonders bevorzugt wird ein pH-Wert von 6 bis 8 eingehalten.

Nach dem Entspannen hält man eine wäßrige Lösung oder Suspension, die monomeres Caprolactam, dessen Oligomere sowie gegebenenfalls nichthydrolysiertes Polycaprolactam enthält. Aus dieser wäßrigen Lösung oder Suspension wird monomeres Caprolactam durch Destillation gewonnen oder wird durch Extrahieren mit einem wasserunlöslichen Lösungsmittel, wie beispielsweise Benzol, Toluol oder Xylol abgetrennt, wobei aus einem solchen Extrakt Caprolactam unschwer durch Abdestillieren des Lösungsmittels erhältlich ist.

Nach einer besonders bevorzugten Arbeitsweise wird bei der Spaltung von Polycaprolactam ein Umsatz von mindestens 60 % eingehalten, aus der erhaltenen wäßrigen Lösung oder Suspension Caprolactam mit wasserunlöslichen organischen Lösungsmitteln extrahiert und die verbleibende wäßrige Lösung oder Suspension, die Oligomere des Caprolactams und gegebenenfalls nicht gespaltenes Polycaprolactam enthält, wieder in die hydrolytische Spaltung zurückgeführt.

Das so erhältliche Caprolactam wird vorteilhaft in die Reinigungsstufe bei der Herstellung von Caprolactam eingeschleust.

Das Verfahren nach der Erfindung sei an folgenden Beispielen veranschaulicht.

### Beispiel 1

In einen 2-Liter Autoklaven wurden 1000 ml Wasser und 100 g Polyamid-6 eingefüllt. Hierzu fügte man 0,5 g Ätznatron. Der Autoklav wurde mit Stickstoff gespült und 5 bar Stickstoff aufgepresst. Anschließend erhöhte man die Temperatur unter Rühren auf 250°C. Der Arbeitsdruck betrug ca. 50 bar. Nach 5 Stunden kühlt man den Autoklaven auf Raumtemperatur ab. Man erhielt eine nahezu farblose klare Lösung.

Nach Extrahieren mit Toluol, wurde das Lösungsmittel durch Destillation aus der organischen Phase entfernt. Als Rückstand erhielt man 67 g Rückstand, aus dem 65 g reines Caprolactam durch Destillation bei 105°C und einem Druck von 4 mbar erhalten wurde.

In der ausextrahierten wäßrigen Phase verblieben 30 g nicht umgesetztes Polyamid.

Das Lactam hatte folgende Qualitätskennzahlen:

| | |
|---|---|
| Permanganattitrationszahl (PTZ) | 52 |
| Extinktion 290 nm einer 50 %igen wäßrigen Lösung, 1 cm Küvettenlänge | 0,19 |
| Flüchtige Basen meq/kg | 0,29 |
| Freie Säuren meq/kg | 0,08 |
| pH-Wert 25 %ige Lösung | 6,5 |
| Verunreinigungen in ppm mittels GC ermittelt | 214 |

### Permanganattitrationszahl (PTZ)

Die Zahl gibt den Verbrauch an 0,1N (0,02 mol/l) Kaliumpermanganatlösung in ml pro kg Caprolactam an. Hierbei titriert man tropfenweise bei 20°C in 50 gew.-%iger Schwefelsäure solange, bis eine schwache Rosafärbung für mindestens 1 Minute bestehen bleibt.

### Vergleichsbeispiel 1

Der Versuch wurde, wie in Beispiel 1 beschrieben, wiederholt. Jedoch wurde dem Autoklavenansatz keine Natronlauge zugesetzt.

Die Aufarbeitung erfolgte, ebenfalls wie beschrieben, durch Toluolextraktion mit nachfolgender Destillation.

| | |
|---|---|
| PTZ | 126 |
| Extinktion 290 nm einer 50 %igen wäßrigen Lösung, 1 cm Küvettenlänge | 0,11 |
| Flüchtige Basen meq/kg | 0,31 |
| Freie Säuren meq/kg | 11,2 |
| pH-Wert | 4,8 |
| Verunreinigungen in ppm mittels GC ermittelt | 381 |

### Beispiel 2

30 g nicht gespaltener Extraktionsrückstand bzw. gebildete Aminocapronsäure aus Beispiel 1 wurde unter den Bedingungen des Beispiels 1 erneut zur Spaltung in den Autoklaven gebracht, 0,5 g Ätznatron hinzugefügt und 5 bar Stickstoff aufgepreßt. Nach einer Verweilzeit von 5 Stunden bei 250°C und 50 bar wurde die wäßrige Lösung extrahiert und aufgearbeitet. Man erhielt 20,5 g Caprolactam mit folgenden Kennzahlen:

| | |
|---|---|
| PTZ | 65 |
| Extinktion 290 nm einer 50 %igen wäßrigen Lösung 1 cm Küvettenlänge | 0,50 |
| Flüchtige Basen meq/kg | 0,80 |
| Freie Säuren meq/kg | 0,1 |
| pH-Wert | 6,2 |
| Verunreinigungen in ppm mittels GC ermittelt | 300 |

## Patentansprüche

1. Verfahren zur Rückgewinnung von Caprolactam aus Polycaprolactam durch hydrolytische Spaltung von Polycaprolactam mit 5 bis 50 Gewichtsteilen Wasser je Gewichtsteil Polycaprolactam unter Zusatz von Alkalihydroxid bei einem pH-Wert von 5 bis 10, einer Temperatur von 200 bis 350°C und einem Druck von 20 bis 100 bar unter Erhalt einer wäßrigen Lösung oder Suspension, die monomeres Caprolactam, dessen Oligomeres und gegebenenfalls Polycaprolactam enthält und anschließendes Abtrennen von monomerem Caprolactam aus der wäßrigen Lösung oder Suspension durch Destillation oder Extraktion.

2. Verfahren nach Anspruch 1, dadurch gekennzeichnet, daß man eine Temperatur von 210 bis 300°C einhält.

3. Verfahren nach den Ansprüchen 1 bis 3, dadurch gekennzeichnet, daß man einen pH-Wert von 6 bis 8 einhält.

4. Verfahren nach den Ansprüchen 1 bis 4, dadurch gekennzeichnet, daß man Natriumhydroxid mitverwendet.

5. Verfahren nach den Ansprüchen 1 bis 5, dadurch gekennzeichnet, daß man eine Verweilzeit bei der hydrolytischen Spaltung von 3 bis 6 Stunden einhält.

6. Verfahren nach den Ansprüchen 1 bis 6, dadurch gekennzeichnet, daß man je Gewichtsteil Polycaprolactam 10 bis 20 Gewichtsteile Wasser anwendet.

7. Verfahren nach den Ansprüchen 1 bis 7, dadurch gekennzeichnet, daß man bei der hydrolytischen Spaltung von Polycaprolactam einen Umsatz von mindestens 60 % einhält, aus der erhaltenen wäßrigen Lösung oder Suspension Caprolactam mit Wasser unlöslichen organischen Lösungsmitteln extrahiert und die verbleibende wäßrige Lösung oder Suspension, die Oligomere des Caprolactams sowie gegebenenfalls nicht gespaltenes Polycaprolactam enthält, wieder in die hydrolytische Spaltung zurückführt.

## Claims

1. A process for the recovery of caprolactam from polycaprolactam by hydrolytic cleavage of polycaprolactam with from 5 to 50 parts by weight of water per part by weight of polycaprolactam with addition of alkali metal hydroxide at a pH of from 5 to 10, a temperature of from 200 to 350°C and a pressure of from 20 to 100 bar, to give an aqueous solution or suspension which contains monomeric caprolactam and oligomers thereof and may contain polycaprolactam and subsequent isolation of monomeric caprolactam from the aqueous solution or suspension by distillation or extraction.

2. A process as claimed in claim 1, wherein a temperature of from 210 to 300°C is maintained.

3. A process as claimed in claim 1 or 2, wherein a pH of from 6 to 8 is maintained.

4. A process as claimed in any of claims 1 to 3, wherein sodium hydroxide is present.

5. A process as claimed in any of claims 1 to 4, wherein a residence time of from 3 to 6 hours is maintained in the hydrolytic cleavage.

6. A process as claimed in any of claims 1 to 5, wherein from 10 to 20 parts by weight of water are used per part by weight of polycaprolactam.

7. A process as claimed in any of claims 1 to 6, wherein a conversion of at least 60% is maintained in the hydrolytic cleavage of polycaprolactam, caprolactam is extracted from the resulting aqueous solution or suspension with a water-insoluble organic solvent and the remaining aqueous solution or suspension, which contains oligomers of caprolactam and may contain uncleaved polycaprolactam, is recycled to the hydrolytic cleavage.

## Revendications

1. Procédé de récupération de caprolactame à partir de polycaprolactame par scission par hydrolyse de polycaprolactame avec 5 à 50 parties en poids d'eau par partie en poids de polycaprolactame, avec addition d'hydroxyde de métal alcalin à une valeur de pH de 5 à 10, à une température de 200 à 350°C et à une pression de 20 à 100 bars, et obtention d'une solution ou suspension aqueuse, qui contient du caprolactame monomère, des oligomères de celui-ci et éventuellement du polycaprolactame, et par isolement ultérieur de caprolactame monomère à partir de la solution ou suspension aqueuse, par distillation ou extraction.

2. Procédé suivant la revendication 1, caractérisé en ce qu'on maintient une température de 210 à 300°C.

3. Procédé suivant l'une des revendications 1 et 2, caractérisé en ce qu'on maintient une valeur de pH de 6 à 8.

4. Procédé suivant l'une des revendications 1 à 3, caractérisé en ce qu'on utilise conjointement de l'hydroxyde de sodium.

5. Procédé suivant l'une des revendications 1 à 4, caractérisé en ce qu'on maintient une durée de séjour pour la scission par hydrolyse de 3 à 6 heures.

6. Procédé suivant l'une des revendications 1 à 5, caractérisé en ce qu'on applique 10 à 20 parties en poids d'eau par partie en poids de polycaprolactame.

7. Procédé suivant l'une des revendications 1 à 6, caractérisé en ce que, au cours de la scission par hydrolyse de polycaprolactame, on maintient un degré de transformation d'au moins 60%, on extrait du caprolactame à partir de la solution ou suspension aqueuse obtenue avec des solvant organiques insolubles dans l'eau et on recycle à nouveau dans la scission par hydrolyse la solution ou suspension aqueuse subsistante, qui contient des oligomères du caprolactame ainsi qu'éventuellement du polycaprolactame non scindé.
